(19) **Europäisches Patentamt**

**European Patent Office**

**Office européen des brevets**

(11) **EP 1 291 439 A2**

(12) **EUROPEAN PATENT APPLICATION**

(43) Date of publication:
**12.03.2003 Bulletin 2003/11**

(51) Int Cl.[7]: **C12Q 1/37**

(21) Application number: **02255715.1**

(22) Date of filing: **15.08.2002**

(84) Designated Contracting States:
**AT BE BG CH CY CZ DE DK EE ES FI FR GB GR
IE IT LI LU MC NL PT SE SK TR**
Designated Extension States:
**AL LT LV MK RO SI**

(30) Priority: **29.08.2001 US 315594**

(71) Applicant: **WARNER-LAMBERT COMPANY
Morris Plains, New Jersey 07950 (US)**

(72) Inventors:
• **Dyer, Richard Dennis,
Pfizer Global Res. and Dev.
Ann Arbor, Michigan 48105 (US)**
• **Hupe, Donald John,
c/o Pfizer Global Res. and Dev.
Ann Arbor, Michigan 48105 (US)**
• **Johnson, Adam Richard,
Pfizer Global Res. and Dev.
Ann Arbor, Michigan 48105 (US)**

(74) Representative: **England, Peter Michael et al
Pfizer Limited,
UK Patent Department,
Ramsgate Road
Sandwich, Kent CT13 9NJ (GB)**

(54) **Method for identifying metalloenzyme inhibitors**

(57) The present invention is a method for identifying a compound as a competitive, noncompetitive, or uncompetitive inhibitor of an enzyme having a functional metal cation, the method comprising assaying the compound for inhibition of the enzyme in the presence of a ligand to the functional metal cation.

**Description**

FIELD OF THE INVENTION

The present invention relates to a method for identifying inhibitors of a metalloenzyme. Such inhibitors are useful for treating mammals that suffer from diseases responsive to inhibition of the metalloenzyme such as arthritis, multiple sclerosis, heart failure, stroke, or cancer, to name a few.

DESCRIPTION OF THE RELATED ART

A metalloenzyme is any naturally occurring mammalian enzyme, or any truncated form thereof, that employs a metal cation directly in a functional capacity, including in a catalytic capacity, as opposed to in a purely structural capacity. Metalloenzymes include matrix metalloproteinases, a disintegrin and metalloproteinase-thrombospondin ("ADAM-TS")-type metalloproteinase such as tumor necrosis factor converting enzyme ("TACE"), alcohol dehydrogenases, carboxypeptidases, alkaline phosphatases, carbonic anhydrases, beta-lactamases, aminopeptidases, Leukotriene-A4 ("LTA4") hydrolases, phospholipases C, *Escherichia coli* ("*E. coli*") and other prokaryotic metallodeacetylases such as UDP-3-*O*-acyl-*N*-acetylglucosamine deacetylase ("LpxC"), prokaryotic peptide deformylase and other iron-dependent enzymes, and isopenicillin N-synthase. Examples of the metal cations employed by such metalloenzymes include $Ca^{2+}$, $Zn^{2+}$, $Co^{2+}$, $Mn^{2+}$, $Mn^{3+}$, $Mg^{2+}$, $Cd^{2+}$, $Ni^{2+}$, $Hg^{2+}$, $Fe^{2+}$, and $Fe^{3+}$.

The matrix metalloproteinases ("MMPs") are naturally occurring (i.e., endogenous) metalloenzymes found in most, if not all, mammals. The catalytic activity of all MMPs is believed to be zinc-dependent. Examples of specific MMPs include collagenase-1 ("MMP-1"), also known as interstitial collagenase; gelatinase A ("MMP-2"); stromelysin-1 ("MMP-3"); matrilysin ("MMP-7"); collagenase-2 ("MMP-8"), also known as neutrophil collagenase; gelatinase B ("MMP-9"); metalloelastase ("MMP-12"); collagenase-3 ("MMP-13"); and membrane-type-1 MMP ("MT1-MMP" or "MMP-14"). Also included are truncated MMP forms, including MMP catalytic domains.

One major biological function of the MMPs is to catalyze the breakdown of connective tissue or extracellular matrix by virtue of their abilities to hydrolyze various components of the tissue or matrix. Examples of the components that may be hydrolyzed by an MMP include collagens (e.g., type I, II, III, or IV), gelatins, proteoglycans, and fibronectins. For example, the collagenase subfamily of MMPs, namely MMP-1, MMP-8, and MMP-13, preferentially cleave collagens, and are thus usually associated with diseases linked to breakdown of collagen-based tissue, for example, arthritis. Another major biological function of the MMPs is proteolytic activation of the zymogen (pro) forms of other MMPs, thereby inducing MMP activation.

Over-expression and/or over-activation of a matrix metalloproteinase ("MMP"), or an imbalance between an MMP and a natural (i.e., endogenous) tissue inhibitor of a matrix metalloproteinase ("TIMP"), has been linked to the pathogenesis of diseases characterized by the breakdown of connective tissue or extracellular matrix. Examples of diseases characterized by over-expression and/or over-activation of an MMP include rheumatoid arthritis; osteoarthritis; osteoporosis; periodontitis; multiple sclerosis; gingivitis; corneal, epidermal, and gastric ulceration; atherosclerosis; neointimal proliferation, which leads to restenosis and ischemic heart failure; stroke; renal disease; macular degeneration; and tumor metastasis.

Further, some MMP-mediated diseases may involve overactivity of only one MMP enzyme. This is supported by the recent discovery that MMP-13 alone is over-expressed in breast carcinoma, while MMP-1 alone is over-expressed in papillary carcinoma.

A drawback of MMP inhibitors identified to date for treatment of the above-listed diseases is their poor selectivity. These MMP inhibitors typically mimic the natural substrates in that they coordinate the functional zinc cation and occupy from 1 to 3 specificity binding pockets along the enzyme active site cleft. As there is much structural similarity among these binding pockets of the various MMPs, this binding mode leads to the poor inhibitor-MMP selectivity.

Another drawback of MMP inhibitors identified to date that coordinate the functional zinc cation of the MMP is that the inhibitors are competitive with substrate binding. As the concentration of an enzyme's substrate rises, the potency of a competitive inhibitor for the active site of the enzyme diminishes. This is a disadvantage for a pharmaceutical agent, as a rising concentration of substrate will eventually reduce the agent's therapeutic efficacy.

A selective MMP inhibitor would avoid potential side effects associated with inhibition of MMPs that are not involved in the pathogenesis of the disease being treated. Further, use of a selective MMP inhibitor would require administration of a lower amount of the inhibitor for treatment of disease than would otherwise be required if the inhibitor was nonselective and, after administration, partitioned in vivo among multiple MMPs. Still further, the administration of a lower amount of compound would improve the margin of safety between the dose of the inhibitor required for therapeutic activity and the dose of the inhibitor at which toxicity is observed.

A noncompetitive or uncompetitive MMP inhibitor would potentially also bind to MMP-TIMP complexes. These inhibitors may not suffer diminishing binding potency in the presence of a rising concentration of substrate. Accordingly, a noncompetitive or uncompetitive MMP inhibitor that binds to an MMP-TIMP complex should maintain its therapeutic efficacy in the presence of the rising substrate concentration. A further advantage of a noncompetitive or uncompetitive MMP inhibitor is that when the inhibitor is bound to an MMP-TIMP complex and the TIMP disassociates from the complex to provide free TIMP and inhibitor-bound MMP, the MMP remains inhibited.

Historically, compounds that are inhibitors of a metalloenzyme have been identified by first mixing a compound, the metalloenzyme, and a substrate of the metalloenzyme; and then determining the degree of inhibition, if any, of the enzyme. These assays cannot be used to identify a compound as a competitive, noncompetitive, or uncompetitive inhibitor.

More particularly, inhibitors of an MMP have been identified by assaying potential inhibitory compounds against the full-length MMP enzyme, or a catalytic domain thereof. These assays are usually performed in the presence of a synthetic substrate of the MMP. The synthetic substrate is cleaved by the MMP in the assay to produce cleavage products, wherein a common property (e.g., ultraviolet absorbance or fluorescence) of the synthetic substrate and/or of one of the cleavage products is measured to determine the extent of cleavage. However, these assays alone do not distinguish between inhibitors that act by binding to the functional metal cation (i.e., competitive inhibitors) and inhibitors that do not bind to the functional metal cation of the target enzyme (i.e., noncompetitive or uncompetitive inhibitors). Rather, first a compound is screened for MMP inhibition; and then laborious and time-consuming enzyme kinetics experiments are run to determine the competitive, noncompetitive, or uncompetitive characteristic of the inhibition. Because enzyme kinetics experiments are so technically-, intellectually-, and time-intensive to perform, very few MMP inhibitors are identified in the scientific literature as competitive, noncompetitive, or uncompetitive inhibitors.

Traditional screening methods for identifying competitive, noncompetitive, or uncompetitive inhibitors of other metalloenzymes have the same drawbacks as the MMP methods. These other screening methods cannot typically distinguish between competitive and noncompetitive inhibitors. Rather, enzyme kinetics experiments are again needed to determine the characteristic of the inhibitor. It can be thus concluded that the same enzyme kinetics methods of identifying competitive, noncompetitive, or uncompetitive inhibitors of other metalloenzymes cannot be used in a high throughput screening ("HTS") mode.

A method of identifying MMP inhibitors that do not coordinate the functional zinc cation which are noncompetitive or uncompetitive inhibitors would thus be valuable. Further, a method of identifying a selective inhibitor of MMP-13 would be particularly valuable, as there appears to be only one reported selective inhibitor of MMP-13, namely WAY-170523.

The present invention provides a method of identifying inhibitors of metalloenzymes, including inhibitors of MMPs that are competitive, noncompetitive, or uncompetitive inhibitors. For example, selective noncompetitive inhibitors of MMP-13 have been identified using the method of the present invention. The method of the present invention works for any enzyme that employs a metal cation in a functional capacity, including in a catalytic mechanism, and is thus applicable to identifying selective inhibitors of any metalloenzyme that acts on a substrate that binds to a metal ion of the enzyme. These and other advantages of this invention will be more fully described in the following text. All that is required to practice the invention method is to determine the relative inhibition of a potential inhibitor versus the metalloenzyme in the absence of the metal binding ligand and in the presence of a suitable concentration of the metal binding ligand.

BRIEF SUMMARY OF THE INVENTION

This invention provides a method for identifying competitive, noncompetitive, or uncompetitive inhibitors of a metalloenzyme. One embodiment of the present invention, hereinafter referred to as Method Embodiment 1, is a method for identifying a compound as a competitive, noncompetitive, or uncompetitive inhibitor of an enzyme having a functional metal cation, the method comprising the steps of:

(a) producing a first control mixture comprising the enzyme, a substrate of the enzyme, and a ligand to the functional metal cation, wherein the first control mixture does not contain the compound;
(b) producing a second control mixture comprising the enzyme and a substrate of the enzyme, wherein the second control mixture does not contain the compound or the ligand to the functional metal cation;
(c) producing a first compound mixture comprising the compound, the enzyme, a substrate of the enzyme, and the ligand to the functional metal cation;
(d) producing a second compound mixture comprising the compound, the enzyme, and a substrate of the enzyme, wherein the second compound mixture does not contain the ligand to the functional metal cation;
(e) determining inhibition of the enzyme in step (c) by comparing the activity of the enzyme in step (c) to the control activity of the enzyme in step (a);
(f) determining inhibition of the enzyme in step (d) by comparing the activity of the enzyme in step (d) to the control activity of the enzyme in step (b); and

(g) comparing the inhibition determined in step (e) with the inhibition determined in step (f).

Another embodiment of the present invention is the method according to Method Embodiment 1, wherein the compound is a competitive inhibitor of the enzyme having a functional metal cation.

Another embodiment of the present invention is the method according to Method Embodiment 1, wherein the compound is a noncompetitive inhibitor of the enzyme having a functional metal cation.

Another embodiment of the present invention is the method according to Method Embodiment 1, wherein the compound is an uncompetitive inhibitor of the enzyme having a functional metal cation.

Another embodiment of the present invention is the method according to Method Embodiment 1, wherein the functional metal cation is selected from: $Ca^{2+}$, $Zn^{2+}$, $Co^{2+}$, $Mn^{2+}$, $Mn^{3+}$, $Mg^{2+}$, $Cd^{2+}$, $Cu^{2+}$, $Ni^{2+}$, $Hg^{2+}$, $Fe^{2+}$, and $Fe^{3+}$.

Another embodiment of the present invention is the method according to Method Embodiment 1, wherein the enzyme having a functional metal cation is selected from: a matrix metalloproteinase, an ADAM-TS type metalloproteinase, including TACE, an alcohol dehydrogenase, a carboxypeptidase, an alkaline phosphatase, a carbonic anhydrase, a beta-lactamase, an aminopeptidase, LTA4 hydrolase, a phospholipase C, a prokaryotic metallodeacetylase, a prokaryotic peptide deformylase, and isopenicillin N-synthase.

Another embodiment of the present invention is the method according to Method Embodiment 1, wherein the enzyme having a functional metal cation is *Escherichia coli* ("*E. coli*") metallodeacetylase.

Another embodiment of the present invention is the method according to Method Embodiment 1, wherein the enzyme having a functional metal cation is LpxC metallodeacetylase.

Another embodiment of the present invention is the method according to Method Embodiment 1, wherein the enzyme having a functional metal cation is a matrix metalloproteinase, or a catalytic domain thereof.

Another embodiment of the present invention is the method according to Method Embodiment 1, wherein the enzyme having a functional metal cation is MMP-1, or a catalytic domain thereof.

Another embodiment of the present invention is the method according to Method Embodiment 1, wherein the enzyme having a functional metal cation is MMP-1 catalytic domain.

Another embodiment of the present invention is the method according to Method Embodiment 1, wherein the enzyme having a functional metal cation is MMP-1 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-2, or a catalytic domain thereof.

Another embodiment of the present invention is the method according to Method Embodiment 1, wherein the enzyme having a functional metal cation is MMP-2 catalytic domain.

Another embodiment of the present invention is the method according to Method Embodiment 1, wherein the enzyme having a functional metal cation is MMP-2 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-3, or a catalytic domain thereof.

Another embodiment of the present invention is the method according to Method Embodiment 1, wherein the enzyme having a functional metal cation is MMP-3 catalytic domain.

Another embodiment of the present invention is the method according to Method Embodiment 1, wherein the enzyme having a functional metal cation is MMP-3 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-7, or a catalytic domain thereof.

Another embodiment of the present invention is the method according to Method Embodiment 1, wherein the enzyme having a functional metal cation is MMP-7 catalytic domain.

Another embodiment of the present invention is the method according to Method Embodiment 1, wherein the enzyme having a functional metal cation is MMP-7 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-8, or a catalytic domain thereof.

Another embodiment of the present invention is the method according to Method Embodiment 1, wherein the enzyme having a functional metal cation is MMP-8 catalytic domain.

Another embodiment of the present invention is the method according to Method Embodiment 1, wherein the enzyme having a functional metal cation is MMP-8 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-9, or a catalytic domain thereof.

Another embodiment of the present invention is the method according to Method Embodiment 1, wherein the enzyme having a functional metal cation is MMP-9 catalytic domain.

Another embodiment of the present invention is the method according to Method Embodiment 1, wherein the enzyme having a functional metal cation is MMP-9 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-12, or a catalytic domain thereof.

Another embodiment of the present invention is the method according to Method Embodiment 1, wherein the enzyme having a functional metal cation is MMP-12 catalytic domain.

Another embodiment of the present invention is the method according to Method Embodiment 1, wherein the enzyme having a functional metal cation is MMP-12 catalytic domain and the ligand to the functional metal cation is 1,10-phenanthroline, or a pharmaceutically acceptable salt thereof.

Another embodiment of the present invention is the method according to Method Embodiment 1, wherein the enzyme having a functional metal cation is MMP-13, or a catalytic domain thereof.

Another embodiment of the present invention is the method according to Method Embodiment 1, wherein the enzyme having a functional metal cation is MMP-13 catalytic domain.

Another embodiment of the present invention is the method according to Method Embodiment 1, wherein the enzyme having a functional metal cation is MMP-13 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is MMP-14, or a catalytic domain thereof.

Another embodiment of the present invention is the method according to Method Embodiment 1, wherein the enzyme having a functional metal cation is MMP-14 catalytic domain.

Another embodiment of the present invention is the method according to Method Embodiment 1, wherein the enzyme having a functional metal cation is MMP-14 catalytic domain, the ligand to the functional metal cation is acetohydroxamic acid, and the inhibitor is a noncompetitive inhibitor.

Another embodiment of the present invention is the method according to Method Embodiment 1, wherein the enzyme having a functional metal cation is a disintegrin and metalloproteinase-thrombospondin-type metalloproteinase.

Another embodiment of the present invention is the method according to Method Embodiment 1, wherein the enzyme having a functional metal cation is tumor necrosis factor converting enzyme.

Another embodiment of the present invention is the method according to Method Embodiment 1, wherein the enzyme having a functional metal cation is an alcohol dehydrogenase.

Another embodiment of the present invention is the method according to Method Embodiment 1, wherein the enzyme having a functional metal cation is a carboxypeptidase.

Another embodiment of the present invention is the method according to Method Embodiment 1, wherein the enzyme having a functional metal cation is an alkaline phosphatase.

Another embodiment of the present invention is the method according to Method Embodiment 1, wherein the enzyme having a functional metal cation is a carbonic anhydrase.

Another embodiment of the present invention is the method according to Method Embodiment 1, wherein the enzyme having a functional metal cation is a beta-lactamase.

Another embodiment of the present invention is the method according to Method Embodiment 1, wherein the enzyme having a functional metal cation is an aminopeptidase.

Another embodiment of the present invention is the method according to Method Embodiment 1, wherein the enzyme having a functional metal cation is a Leukotriene-A4 hydrolase.

Another embodiment of the present invention is the method according to Method Embodiment 1, wherein the enzyme having a functional metal cation is a phospholipase C.

Another embodiment of the present invention is the method according to Method Embodiment 1, wherein the enzyme having a functional metal cation is a prokaryotic peptide deformylase.

Another embodiment of the present invention is the method according to Method Embodiment 1, wherein the enzyme having a functional metal cation is isopenicillin N-synthase.

Another embodiment of the present invention is the method according to Method Embodiment 1, wherein the ligand to the functional metal cation is acetohydroxamic acid.

Another embodiment of the present invention is the method according to Method Embodiment 1, wherein the ligand to the functional metal cation is 1,10-phenanthroline, or a pharmaceutically acceptable salt thereof.

Another embodiment of the present invention is the method according to Method Embodiment 1, further comprising utilizing steady-state kinetics to determine whether the inhibitor is a noncompetitive inhibitor or uncompetitive inhibitor.

BRIEF DESCRIPTION OF THE FIGURE

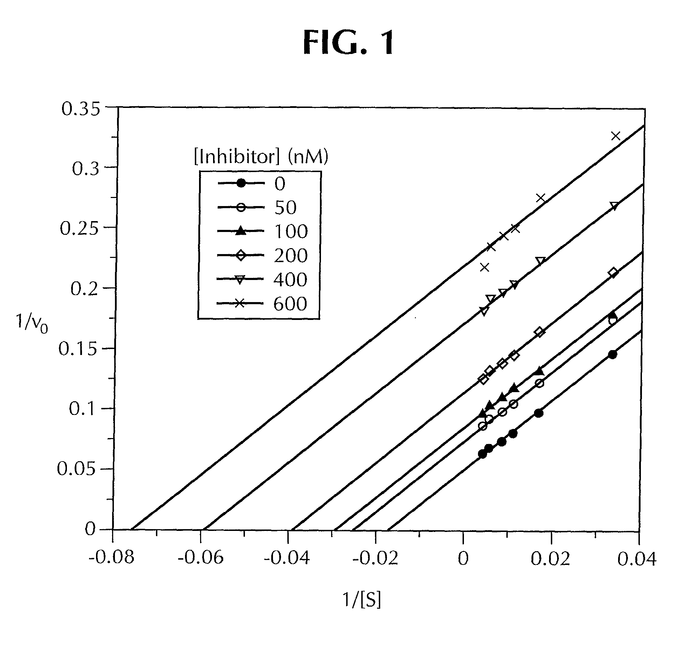

Figure 1 is a graph that plots 1 over the initial velocity $V_O$, which is the initial rate of product formation in a steady state kinetics assay, on the Y-axis versus 1 over concentration of substrate [S] used in the assay on the X-axis for inhibition of MMP-2 with N-[(3-phenylisoxazol-5-ylmethyl)-aminothiocarbonyl]-benzamide at 6 concentrations of from 0 nanomolar to 600 nanomolar.

DETAILED DESCRIPTION OF THE INVENTION

As described above, the present invention is a method for identifying a compound as a competitive, noncompetitive, or uncompetitive inhibitor of an enzyme having a functional metal cation, the method comprising the steps of:

(a) producing a first control mixture comprising the enzyme, a substrate of the enzyme, and a ligand to the functional metal cation, wherein the first control mixture does not contain the compound;
(b) producing a second control mixture comprising the enzyme and a substrate of the enzyme, wherein the second control mixture does not contain the compound or the ligand to the functional metal cation;
(c) producing a first compound mixture comprising the compound, the enzyme, a substrate of the enzyme, and the ligand to the functional metal cation;
(d) producing a second compound mixture comprising the compound, the enzyme, and a substrate of the enzyme, wherein the second compound mixture does not contain the ligand to the functional metal cation;
(e) determining inhibition of the enzyme in step (c) by comparing the activity of the enzyme in step (c) to the control activity of the enzyme in step (a);
(f) determining inhibition of the enzyme in step (d) by comparing the activity of the enzyme in step (d) to the control activity of the enzyme in step (b); and
(g) comparing the inhibition determined in step (e) with the inhibition determined in step (f).

It should be appreciated that it is not necessary to use the same lot of metalloenzyme in steps (a), (b), (c), or (d) above, or to perform steps (a), (c), and (e) at or about the same time that steps (b), (d), and (f) are performed. However, it should be appreciated that the specific activity of a particular lot of a metalloenzyme may be different from the specific activity of another lot of the enzyme due to variations in conditions between the preparation of one lot of metalloenzyme versus the preparation of the other lot of metalloenzyme. Accordingly, use of the same lot of metalloenzyme in steps (a), (b), (c), and (d) and performance of steps (a), (c), and (e) at or about the same time as performance of steps (b), (d), and (f) is preferred.
As used herein, the term "metalloenzyme" means any full-length mammalian enzyme, or a truncated form or a catalytic domain thereof, that employs a metal cation in a functional capacity, as opposed to a structural capacity. That is, a functional metal cation coordinates directly via a noncovalent bond, or indirectly via, for example, a bridging water molecule or a conjugate acid or conjugate base form thereof, to a functional group of the substrate being acted upon by the enzyme. The substrate may be, for example, an amino acid, an alcohol, a peptide, molecular oxygen (e. g., in the case of certain iron-containing enzymes such as hemoglobin), a nucleotide, a polynucleotide, carbon dioxide (e.g., carbonic anhydrase), a coenzyme, and the like. The functional metal cation of the metalloenzyme may, or may not, be an active-site metal cation.
It should be appreciated that the term "metalloenzyme" is synonymous with the phrase "enzyme having a functional metal cation".
The phrase "catalytic domain" means the domain containing the functional metal cation of a metalloenzyme, wherein the metalloenzyme contains two or more domains. A catalytic domain includes truncated forms thereof. For example, the collagenases, of which MMP-13 is a member, have been reported to contain a signal peptide domain, a propeptide domain, a catalytic domain, and a hemopexin-like domain (Ye Qi-Zhuang, Hupe D., Johnson L., *Current Medicinal Chemistry,* 1996;3:407-418).
The phrase "competitive inhibitor" means an inhibitor of an enzyme having a functional metal cation wherein the inhibitor competes with a ligand to the functional metal cation for binding to said cation. Binding of a competitive inhibitor or the ligand to the functional metal cation of a metalloenzyme are mutually exclusive events. It should be appreciated that the potency of a competitive inhibitor decreases as the concentration of substrate increases.
The phrase "noncompetitive inhibitor" means an inhibitor of an enzyme having a functional metal cation, wherein the inhibitor may bind to the enzyme, to a metalloenzyme-substrate complex, or to a metalloenzyme-product complex. A noncompetitive inhibitor does not compete with a ligand to the functional metal cation for binding to said cation, and thus does not prevent binding of a substrate or a product to a metalloenzyme. A noncompetitive inhibitor acts by decreasing the turnover number of an enzyme (i.e., the number of substrate molecules converted to product by the enzyme, per unit time, when the enzyme is fully saturated with substrate).

A noncompetitive inhibitor acts by binding to a metalloenzyme, a metalloenzyme-substrate complex, or a metalloenzyme-product complex without binding to a functional metal cation of the metalloenzyme. The binding affinities of the noncompetitive inhibitor for the metalloenzyme and the metalloenzyme-substrate complex, or the metalloenzyme and the metalloenzyme-product complex, may be the same or different. A noncompetitive inhibitor that has different binding affinities for the metalloenzyme and the metalloenzyme-substrate complex, or the metalloenzyme and the metalloenzyme-product complex, is known as a "mixed" inhibitor.

The phrase "uncompetitive inhibitor" means an inhibitor of an enzyme having a functional metal cation, wherein the inhibitor binds to the enzyme-substrate complex only. An uncompetitive inhibitor does not compete with the ligand to the functional metal cation for binding to the functional metal cation of the metalloenzyme-substrate complex.

Characterization of an inhibitor by the instant method as competitive, noncompetitive, or uncompetitive may be readily confirmed, if necessary or desired, by one skilled in the art by performing conventional steady-state kinetics experiments.

The term "compound" includes the free acid or free base of a compound, or a pharmaceutically acceptable salt thereof, and further includes solvates, including hydrates, any stereoisomer thereof, including (R) and (S), any diastereomer, enantiomer, and epimer, and mixtures thereof, any geometric isomer thereof, including cis, trans, syn, anti, entgegen (E), and zusammen (Z) isomers, if any, and any tautomeric form thereof, including an enolic form of a carbonyl that is proximal to an alpha hydrogen atom.

The phrase "ligand to the functional metal cation" means any compound that binds to the functional metal cation of an enzyme having a functional metal cation. The binding may be of a direct or indirect character. Indirect binding of a ligand via a noncovalent bond to the functional metal cation includes coordination via a bridging water molecule or conjugate acid/base thereof.

In principle, a ligand of any potency, concentration, or size will work in the method of the instant invention provided that the ligand is at a concentration such that, in the assay mixture, some unbound metalloenzyme is present and some bound metalloenzyme is present such that catalytic activity of the enzyme is detectable by the assay method employed, and the graded inhibition of the enzyme activity, in the presence or absence of the ligand, is differentiable. Although any size ligand will work in the invention method, a large ligand will block more binding sites of the metalloenzyme than a smaller ligand will block, and may thus prevent potential inhibitors that bind close to, but not at, the functional metal cation from being identified by the invention method.

In any of the embodiments of the instant invention described above, a preferred ligand to the functional metal cation is a low molecular weight (i.e., a molecular weight of from 30 to 750 atomic units) molecule. A more preferred ligand has a molecular weight of from 40 to 500 atomic units. A still more preferred ligand has a molecular weight of from 50 to 250 atomic units.

Illustrative examples of the ligand to a functional metal cation include acetohydroxamic acid, acetic acid, propanoic acid, N-hydroxy-propanamide, acetoacetic acid, malonic acid, ethanethiol, 1,3-propanedithiol, N-hydroxy-benzamide, imidazole, 2-mercaptoethanol, cyanide, thiocyanate, 2,4,6-trihydroxypyrimidine, 1,10-phenanthroline, and the like. Low molecular weight, known inhibitors of a particular metalloenzyme such as, for example, a dipeptide inhibitor may be used as the ligand to the functional metal cation.

In any of the embodiments of the instant invention described above, preferred is a concentration of the ligand of from about 0.5 to about 3 $K_d$, wherein "$K_d$" is the disassociation constant for the ligand-enzyme complex. One $K_d$ is equal to the $K_i$ of the ligand with the enzyme. More preferred is a concentration of the ligand that is from about 1 $K_d$ to about 2 $K_d$. A concentration of the ligand that is about 1 $K_d$ means that about 50% of the target enzyme is bound to the ligand and about 50% of the target enzyme is free (i.e., unbound). In any of the embodiments of the instant invention described above, preferred is a concentration of the ligand that provides from about 20% bound target enzyme to about 90% bound target enzyme. More preferred is a concentration of the ligand that provides from about 35% bound target enzyme to about 90% bound target enzyme. Still more preferred is a concentration of the ligand that provides from about 40% bound target enzyme to about 90% bound target enzyme.

The term "about", when used herein to modify a number, means a numerical range that includes all values that may be rounded to the number being modified by the term. For example, in the above phrase "about 1 $K_d$ to about 2 $K_d$", about 1 $K_d$ means $K_d$ in a range of from 0.5 to <1.5, which includes 0.5, 0.50001, and 1.4999999999. Each of 0.5, 0.50001, or 1.4999999999 may be rounded to the number being modified by about, which number is 1. Likewise, about 2 $K_d$ means $K_d$ in a range of from 1.5 to <2.5, and includes 1.5, 1.50001, and 2.4999999999. Accordingly, the phrase "about 1 $K_d$ to about 2 $K_d$" includes $K_d$ values in the range of from 0.5 to 2.49. Further examples include "about 50%," which means a range of from 45% to <55%, and about pH 7.4, which means a range of from pH 7.35 to <pH 7.45.

The term "$IC_{50}$" means the concentration of inhibitor required to inhibit the activity of an enzyme having a functional metal cation by 50% compared to the activity of the uninhibited metalloenzyme or to the activity of the metalloenzyme inhibited by a ligand to the functional metal cation.

The phrase "acetohydroxamic acid" means $CH_3C(O)N(H)OH$, which is synonymous with $CH_3CONHOH$, Ac-NHOH, and $AcN(H)OH$.

The term "mammal" includes a human, cow, horse, pig, dog, cat, sheep, rat, mouse, rabbit, and guinea pig.

The term "comprising", which is synonymous with the terms "including," "containing," or "characterized by" is inclusive or open-ended, and does not exclude additional, unrecited elements or method steps from the scope of the invention that is described following the term.

The phrase "consisting of" is closed-ended, and excludes any element, step, or ingredient not specified in the description of the invention that follows the phrase.

The phrase "consisting essentially of" limits the scope of the invention that follows to the specified elements, steps, or ingredients, and those further elements, steps, or ingredients that do not materially affect the basic and novel characteristics of the invention.

The method of the present invention is useful for identifying a compound, or a pharmaceutically acceptable salt thereof, as a competitive, noncompetitive, or uncompetitive inhibitor of a metalloenzyme. Pharmaceutically acceptable salts, include, but not limited to, acid addition and/or base salts. The acid addition salts are formed from basic compounds, whereas the base addition salts are formed from acidic compounds. All of these forms are within the scope of the compounds that may be identified as a competitive, noncompetitive, or uncompetitive inhibitor of a metalloenzyme by the method of the present invention.

Pharmaceutically acceptable acid addition salts of the basic compounds identified according to the method of the present invention include nontoxic salts derived from inorganic acids such as hydrochloric, nitric, phosphoric, sulfuric, hydrobromic, hydroiodic, hydrofluoric, phosphorous, and the like, as well as nontoxic salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, malate, tartrate, methanesulfonate, and the like. Also contemplated are salts of amino acids such as arginate, and the like, glucosamine, gluconate, and galacturonate (see, for example, Berge S.M. et al., "Pharmaceutical salts," *J. of Pharma. Sci.,* 1977;66:1). All of these forms are within the scope of the . compounds that may be identified by the method of the present invention.

Further, the invention method works for any form of the compound, including unsolvated forms as well as solvated forms, including hydrated forms; any (R) or (S), diastereomeric, enantiomeric, and epimeric form of the compound, if any, as well as mixtures thereof; all cis, trans, syn, anti, and entgegen (E), and zusammen (Z) isomers, if any, as well as mixtures thereof; all tautomeric forms, if any, including those tautomeric forms that interchange, for example, via enolization/de-enolization, and the like.

As described above, the invention method employs a ligand to the functional metal cation of a metalloenzyme. The ligand may be readily identified by screening compounds, preferably low molecular weight compounds, against the particular metalloenzyme employed in the presence of a suitable enzyme substrate. This screening may be readily accomplished using a conventional art-recognized assay method for the particular enzyme being employed. Acetohydroxamic acid may be used as the ligand.

As mentioned above, the method of the present invention may be used to identify a competitive, noncompetitive, or uncompetitive inhibitor of any metalloenzyme. Such identification is to compare the inhibition of the metalloenzyme by a compound in the absence of the ligand to the functional metal cation, to the inhibition of the metalloenzyme by the compound in the presence of the ligand to the functional metal cation. This comparison can be, for example, by calculating a ratio of inhibition. One method of comparing is to determine, by conventional means, the inhibition as $IC_{50}$ values, respectively, and to calculate an $IC_{50}$ value ratio as the $IC_{50}$ value of the compound with the metalloenzyme in the presence of the ligand divided by the $IC_{50}$ value of the compound in the presence of the ligand. If the $IC_{50}$ value ratio is <1, the inhibitor is noncompetitive or uncompetitive, and the inhibitor is synergistic with the ligand to the functional metal. If the ratio is equal to I, the inhibitor is noncompetitive. On the other hand, if the ratio is >1, the inhibitor is competitive.

There are many other ways of determining the inhibition ratio such as, for example, by dividing the inhibition of a compound, at a known concentration, in the presence of the ligand by the inhibition of the compound, at the same concentration, in the absence of the ligand. Another example of determining the inhibition ratio is dividing the percent inhibition of a compound at a known concentration in the presence of the ligand by the percent inhibition of the compound at the same concentration in the absence of the ligand. Enzyme kinetics experiments may be further employed, when necessary, as part of the invention method to differentiate between competitive, noncompetitive, and uncompetitive inhibitors.

In practice, an assay for a metalloenzyme is carried out with and without a test compound, and the rates of hydrolysis, as indicated by the steady-state initial reaction velocities of cleavage of substrate by the metalloenzyme, are measured to determine inhibitory activity of the test compound. Initial reaction velocities of cleavage of substrate by the metalloenzyme may be determined by measuring the rate of substrate cleavage or the rate of reaction product formation. Measurements may be made utilizing spectrophotometric means, fluorimetric means, or by SDS-polyacrylamide gel electrophoresis ("SDS-PAGE").

For example, when fluorimetric means are utilized, changes in absorbance without a test compound and with test compound at different concentrations of compound such as, for example, 100, 10, and 1 μM, or 100, 10, and I nM may be measured. Alternatively when fluorimetric means are utilized, changes in fluorescence may be measured by comparing fluorescence without a test compound to fluorescence with a test compound, wherein the comparisons are performed at different concentrations of test compound, such as those described immediately above. The compound concentration is then plotted on the X-axis against the percentage of control activity observed for experiments with compound versus experiments without compound (i.e., (velocity with a ligand to the functional metal cation) divided by (velocity without a ligand to the functional metal cation) x 100) on the Y-axis to define $IC_{50}$ values. These experiments are run both in the presence and the absence of the ligand to the functional metal cation. Data are fit to the equation:

$$\%control\ activity = 100/[1+(([I]/IC_{50})^{slope})],$$

where [I] is the compound concentration, $IC_{50}$ is the concentration of compound where the reaction rate is 50% inhibited relative to the control reaction, and slope is the slope of the $IC_{50}$ curve at the curve's inflection point, using nonlinear least-squares curve fitting equation regression.

Alternatively, the inhibition ratio may be determined by comparing the amount of substrate cleavage or reaction product formation at a single time point (e.g., 30 minutes after addition of compound), measured by spectrophotometric means, fluorimetric means, or SDS-PAGE, to the initial amount of substrate or reaction product for a compound in the presence of a ligand to the functional metal cation, and then in the absence of a ligand to the functional metal cation.

The assay method, and substrate employed therein, for a particular metalloenzyme is a method and substrate known in the art to be useful for screening for inhibitors of the metalloenzyme. A ligand to the functional metal cation of the metalloenzyme is first identified by screening compounds for inhibition of the metalloenzyme using conventional means. Preferably, the $K_i$ of the ligand with the metalloenzyme is determined by conventional means, and the concentration of ligand employed in the invention method is about equal to the $K_i$ value, or within the range of $K_d$ as described above.

All that is needed to practice the invention method for any particular enzyme is to employ the ligand to the functional metal cation of the metalloenzyme in an art-recognized assay at or about the time the substrate is employed, according to the guidelines described above. The ligand may be first buffered to the assay pH before it is added to the assay mixture. The invention method may be readily adapted for use in high throughput screening mode by a person of ordinary skill in the art by following conventional means of adaptation.

For example, the method of the present invention for identifying a competitive, noncompetitive, or uncompetitive inhibitor of an MMP may be carried out as follows below in the examples. Some of the particular methods described below use the catalytic domain of the MMP-13 enzyme, namely matrix metalloproteinase-13 catalytic domain ("MMP-13CD"), rather than the corresponding full-length enzyme, MMP-13. It has been shown previously by Ye Qi-Zhuang, Hupe D., and Johnson L. (*Current Medicinal Chemistry,* 1996;3:407-418) that inhibitor activity against a catalytic domain of an MMP is predictive of the inhibitor activity against the respective full-length MMP enzyme.

The following examples are provided merely to further illustrate the invention. The scope of the invention is not to be construed as merely consisting of the following examples.

**EXAMPLE 1**

**Thiopeptolide substrate-based assay for identifying competitive, noncompetitive, or uncompetitive inhibitors of stromelysin-1 catalytic domain ("MMP-3CD"):**

A standard assay for measuring the amount by which a test compound inhibits the hydrolysis of a thiopeptolide ("TPL") substrate caused by a representative matrix metalloproteinase enzyme, namely MMP-3 catalytic domain ("MMP-3CD"), is described in detail by Ye, Q-Z. et. al., in *Biochemistry,* 1992;31(45):11231-11235, which is incorporated by reference herein. An amount of the ligand to the functional metal cation such as, for example, 1 $K_d$ equivalent of acetohydroxamic acid, may be added to the assay, which is described below, to provide a method of the present invention.

Thiopeptolide substrates show virtually no decomposition or hydrolysis in the absence of a matrix metalloproteinase enzyme at pH <7.6. A typical thiopeptolide substrate commonly utilized for assays that determine inhibition of an MMP is Ac-Pro-Leu-Gly-thioester; -Leu-Leu-Gly-Oet. For example, for MMP-3CD, a 100 μL assay mixture will

contain 50 mM of 2-morpholinoethane-sulfonic acid monohydrate buffer (MES, pH 6.0), 10 mM $CaCl_2$, 100 µM thiopeptolide substrate, acetohydroxamic acid, and 1 mM 5,5'-dithio-bis(2-nitrobenzoic acid) ("DTNB"). The thiopeptolide substrate concentration is varied from 10 µM to 800 µM to obtain $K_m$ and $K_{cat}$ values. The change in absorbance at 405 nm is monitored on a THERMO MAX microplate reader (Molecular Devices, Menlo Park, CA) at room temperature (22°C typically). The calculation of the amount of hydrolysis of the thiopeptolide substrate is based on $E_{412} = 13600$ $m^{-1}cm^{-1}$ for the DTNB-derived product 3-carboxy-4-nitrothiophenoxide. In practice, assays are carried out with and without an MMP inhibitor test compound, and the rates of hydrolysis, as indicated by the steady-state initial velocities, are compared to determine inhibitory activity of the test compound.

Initial reaction velocities of thiopeptolide cleavage are determined by measuring the changes in absorbance without an MMP inhibitor test compound and with an MMP inhibitor test compound at different concentrations such as, for example, 100, 10, and 1 µM, or 100, 10, and 1 nM. The inhibitor concentration is then plotted on the X-axis against the percentage of control activity observed for inhibited experiments versus uninhibited experiments (i.e., (velocity with inhibitor) divided by (velocity without inhibitor) $\times$ 100) on the Y-axis to define $IC_{50}$ values. These experiments are run both in the presence and the absence of acetohydroxamic acid. Data are fit to the equation: %control activity = 100/ $[1+(([I]/IC_{50})^{slope})]$, where [I] is the MMP inhibitor test compound concentration, $IC_{50}$ is the concentration of MMP inhibitor test compound where the reaction rate is 50% inhibited relative to the control reaction, and slope is the slope of the $IC_{50}$ curve at the curve's inflection point, using nonlinear least-squares curve fitting equation regression.

## EXAMPLE 2

**Fluorigenic peptide-1 substrate based assay for identifying competitive, noncompetitive, or uncompetitive inhibitors of MMP-13CD:**

**Final assay conditions:**

    50 mM HEPES buffer (pH 7.0)
    10 mM $CaCl_2$
    10 µM fluorigenic peptide-1 ("FP1") substrate
    0 or 15 mM acetohydroxamic acid (AcNHOH) = 1 $K_d$
    2% DMSO (with or without inhibitor test compound)
    0.5 nM MMP-13CD enzyme

**Stock solutions:**

    (1) <u>10X assay buffer</u>: 500 mM HEPES buffer (pH 7.0) plus 100 mM $CaCl_2$
    (2) <u>10 mM FP1 substrate</u>: (Mca)-Pro-Leu-Gly-Leu-(Dnp)-Dpa-Ala-Arg-$NH_2$ (Bachem, M-1895; "A novel coumarin-labeled peptide for sensitive continuous assays of the matrix metalloproteinases," Knight C.G., Willenbrock F., and Murphy, G., *FEBS Lett.,* 1992;296:263-266). Prepared 10 mM stock by dissolving 5 mg FP1 in 0.457 mL DMSO.
    (3) <u>3 M AcNHOH:</u> Prepared by adding 4 mL $H_2O$ and 1 mL 10X assay buffer to 2.25 g AcNHOH (Aldrich 15,903-4). Adjusted pH to 7.0 with NaOH. Diluted volume to 10 mL with $H_2O$. Final solution contained 3 M AcNHOH, 50 mM HEPES buffer (pH 7.0), and 10 mM $CaCl_2$.
    (4) <u>AcNHOH dilution buffer:</u> 50 mM HEPES buffer (pH 7.0) plus 10 mM $CaCl_2$
    (5) <u>MMP-13CD enzyme:</u> Stock concentration = 250 nM.
    (6) <u>Enzyme dilution buffer:</u> 50 mM HEPES buffer (pH 7.0), 10 mM $CaCl_2$, and 0.005% BRIJ 35 detergent (Calbiochem 203728; Protein Grade, 10%)

**Procedure (for one 96-well microplate):**

A. *Prepared assay mixture:*

    1100 µL 10X assay buffer
    11 µL 10 mM FP1

55 µL 3 M AcNHOH or 55 µL AcNHOH dilution buffer
8500 µL H$_2$O

*B. Diluted MMP-13CD to 5 nM working stock:*

22 µL MMP-13CD (250 nM)
1078 µL enzyme dilution buffer

*C. Ran kinetic assay:*

1. Dispensed 2 µL inhibitor test sample (in 100% DMSO) into well.
2. Added 88 µL assay mixture and mixed well, avoiding bubbles.
3. Initiated reactions with 10 µL of 5 nM MMP-13CD; mixed well, avoiding bubbles.
4. Immediately measured the kinetics of the reactions at room temperature. Fluorimeter: F$_{max}$ Fluorescence Microplate Reader & SOFTMAX PRO Version 1.1 software (Molecular Devices Corporation; Sunnyvale, CA 94089).

| Protocol menu: | |
|---|---|
| excitation: 320 nm | emission: 405 nm |
| run time: 15 min | interval: 29 sec |
| RFU min: -10 | RFU max: 200 |
| $V_{max}$ points: 32/32 | |

*D. Compared % of control activity and/or IC$_{50}$ with inhibitor test compound ±AcNHOH*

Hydrolysis of the fluorigenic peptide-1 substrate, [(Mca)Pro-Leu-Gly-Leu-Dpa-Ala-Arg-NH$_2$; Bachem, catalog number M-1895], wherein "Mca" is (7-methoxy-coumarin-4-yl)acetyl and "Dpa" is (3-[2,4-dinitrophenyl]-L-2,3-diaminopropionyl), was used to screen for MMP-13 catalytic domain (CD) inhibitors. (Dpa may also be abbreviated as "Dnp".) Reactions (100 µL) contained 0.05 M Hepes buffer (pH 7), 0.01 M calcium chloride, 0.005% polyoxyethylene (23) lauryl ether ("Brij 35"), 0 or 15 mM acetohydroxamic acid, 10 µM FP1, and 0.1 mM to 0.5 nM inhibitor in DMSO (2% final).
After recombinant human MMP-13CD (0.5 nM final) was added to initiate the reaction, the initial velocity of FP1 hydrolysis was determined by monitoring the increase in fluorescence at 405 nm (upon excitation at 320 nm) continuously for up to 30 minutes on a microplate reader at room temperature. Alternatively, an endpoint read can also be used to determine reaction velocity provided the initial fluorescence of the solution, as recorded before addition of enzyme, is subtracted from the final fluorescence of the reaction mixture. The inhibitor was assayed at different concentration values, such as, for example, 100, 10, and 1 µM, or 100, 10, and 1 nM. Then the inhibitor concentration was plotted on the X-axis against the percentage of control activity observed for inhibited experiments versus uninhibited experiments (i.e., (velocity with inhibitor) divided by (velocity without inhibitor) $\times$ 100) on the Y-axis to determine IC$_{50}$ values. This determination was done for experiments done in the presence, and experiments done in the absence, of acetohydroxamic acid. Data were fit to the equation: %control activity = $100/[1+(([I]/IC_{50})^{slope})]$, where [I] is the inhibitor concentration, IC$_{50}$ is the concentration of inhibitor where the reaction rate is 50% inhibited relative to the control, and slope is the slope of the IC$_{50}$ curve at the curve's inflection point, using nonlinear least-squares curve-fitting equation regression.
Results of Example 2 are shown below in Table 1 in the column labeled "IC$_{50}$ Ratio (±)". In Table 1, noncompetitive or uncompetitive inhibitors that have an IC$_{50}$ Ratio (±) ratio of <1 are synergistic with AcNHOH, while competitive inhibitors have an IC$_{50}$ Ratio (±) of >1, unless otherwise indicated.

Table 1

| Entry No. | Compound Tested | Without AcNHOH $IC_{50}$ ($\mu M$) | With AcNHOH $IC_{50}$ ($\mu M$) | $IC_{50}$ Ratio ($\pm$) |
|---|---|---|---|---|
| 1 | | 34 | 16 | 0.47[a] |
| 2 | | 84 | 3.1 | 0.04 |
| 3 | | 126 | 71 | 0.56 |
| 4 | | 64 | 4.7 | 0.07[a] |
| 5 | | 9.5 | 11 | 1.16 |
| 6 | | 17 | 14 | 0.82 |

[a]  Kinetics experiments (not shown) demonstrate these compounds are noncompetitive inhibitors of MMP-13CD.

12

## TABLE 1
### (Cont'd)

| Entry No. | Compound Tested | Without AcNHOH IC$_{50}$ ($\mu$M) | With AcNHOH IC$_{50}$ ($\mu$M) | IC$_{50}$ Ratio ($\pm$) |
|---|---|---|---|---|
| 7 | | 27 | 11 | 0.41 |
| 8 | | 11 | 5.2 | 0.47 |
| 9 | | 0.19 | 0.07 | 0.37[a] |
| 10 | | 2.3 | 0.2 | 0.08 |

[a]  Kinetics experiments (not shown) demonstrate these compounds are noncompetitive inhibitors of MMP-13CD.

**EXAMPLE 3**

**Fluorigenic peptide-1 based assay for identifying competitive, noncompetitive, or uncompetitive inhibitors of matrix metalloproteinase-13 catalytic domain ("MMP-13CD"):**

In a manner similar to Example 2, an assay is run wherein 1,10-phenanthroline is substituted for acetohydroxamic acid to identify a competitive, noncompetitive, or uncompetitive inhibitors of MMP-13CD.

**EXAMPLE 4**

**Screening assay for MMP-12 inhibitors**

**Materials and Methods**

**Enzyme and reagents**: Human MMP-12 catalytic domain ("MMP-12CD") was cloned, expressed in E. coli and purified using a denaturation/renaturation method. A fluorigenic petide-1 (FP-1) with the sequence: Mca-Pro-Leu-Gly-Leu-Dap(Dnp)-Ala-Arg-NH$_2$ was purchased from Bachem (ref: M-1895). Stock solution was prepared in DMSO at 10 mM and kept at -20°C. All the other reagents were from Sigma.
**Plate preparation:** For screening, 4 μL of the compounds were added to 384-well black microplates at 250 μM in 25% DMSO. For IC$_{50}$ determination, a range of 8 dilutions were prepared in 25% DMSO, and 4 μL of each concentration were added to the plates in duplicates.
**Assay:** The reaction was started by sequential addition of 41 μL of the FP-1 (10 μM final concentration) in assay buffer (50 mM Tris-HCl, 10 mM CaCl$_2$) containing 5 mM AcNHOH and 5 μL of enzyme diluted in assay buffer containing 0.005% Brij-35. The microplates were incubated for 20 minutes at room temperature. The fluorescence changes were recorded in a Fluostar (BMG) instrument using excitation filter at 320 nm and emission filter at 405 nm. Results in wells containing compounds were calculated as a percentage of the fluorescence signal in control wells that received aqueous DMSO, but no compound (maximum signal), or no enzyme (minimum signal). **Results:** The signal to background ratio was between 5 to 8 with Z' value (a quality control factor, see JI-HU ZHANG et. al., *Journal of Biomolecular Screening,* 1999;4(2):67-73) in screening higher than 0.4% on each plate. IC$_{50}$ data were processed in a manner similar to that described for Example 2 to identify competitive, noncompetitive, or uncompetitive inhibitors of MMP-12CD.

**EXAMPLE 5**

**Fluorigenic peptide-2 based assay for identifying competitive, noncompetitive, or uncompetitive inhibitors of tumor necrosis factor converting enzyme ("TACE")**

In a manner similar to Example 2, an assay is run using about I $K_d$ of acetohydroxamic acid (determined with TACE), wherein TACE is substituted for MMP-13CD and Fluorigenic peptide-2 ("FP-2"). Mca-Pro-Leu-Ala-Gln-Ala-Val-Dap(Dnp)-Arg-Ser-Ser-Ser-Arg-NH$_2$) is substituted for FP-1 to identify a competitive, noncompetitive, or uncompetitive inhibitor of TACE.

**EXAMPLE 6**

**Fluorigenic peptide-1 substrate based assay for identifying competitive, noncompetitive or uncompetitive inhibitors of MMP-2**

In a manner similar to Example 2, an assay is run using about 1 $K_d$ of acetohydroxamic acid (determined with MMP-2), wherein MMP-2 is substituted for MMP-13CD, to identify a competitive, noncompetitive, or uncompetitive inhibitor of MMP-2.

**EXAMPLE 7**

**Fluorigenic peptide-1 substrate based assay for identifying competitive, noncompetitive, or uncompetitive inhibitors of MMP-9**

In a manner similar to Example 2, an assay is run using about 1 $K_d$ of acetohydroxamic acid (determined with MMP-9), wherein MMP-9 is substituted for MMP-13CD, to identify a competitive, noncompetitive, or uncompetitive inhibitor of MMP-9.

Results from Examples 2, 6, and 7 for N-[(3-phenylisoxazol-5-ylmethyl)-aminothiocarbonyl]-benzamide are shown below in Table 2 in the column labeled "IC$_{50}$ Ratio ($\pm$)." In Table 2, noncompetitive or uncompetitive inhibitors that have an IC$_{50}$ Ratio ($\pm$) of less than 1 are synergistic with AcNHOH, which competitive inhibitors have an IC$_{50}$ Ratio ($\pm$) of greater than 1, unless otherwise indicated.

### Table 2. Synergistic Inhibition of MMP-2, MMP-9, and MMP-13CD

| MMP | Without AcNHOH IC$_{50}$ ($\mu$M) | With AcNHOH IC$_{50}$ ($\mu$M) | IC$_{50}$ Ratio ($\pm$) |
|---|---|---|---|
| 2FL | 0.9 | 0.09 | 0.10 |
| 9FL | 3.1 | 1.2 | 0.39 |
| 13CD | 2.3 | 0.2 | 0.09 |

\* Fluorigenic peptide (FP1) substrate assay

Steady-state kinetics of the mechanism of inhibition of MMP-2 by the compound of Table 2 are shown in Figure 1, and show the compound is an uncompetitive inhibitor of MMP-2. In Figure 1, 1 over the initial velocity V$_O$, which is the initial rate of product formation in a steady state kinetics assay, is plotted on the Y-axis versus 1 over concentration of substrate [S] used in the assay on the X-axis for inhibition of MMP-2 with N-[(3-phenylisoxazol-5-ylmethyl)-aminothiocarbonyl]-benzamide at 6 concentrations of from 0 nanomolar to 600 nanomolar.

In principle, the method of Examples 1 to 4 may be readily adapted to identify competitive, noncompetitive, or uncompetitive inhibitors of any MMP, or a catalytic domain thereof, by substituting for MMP-13CD and the substrate of Example 1 or 2, the particular MMP, or a catalytic domain thereof, being screened against and an art-recognized substrate of the particular MMP, respectively. For example, the method has been adapted to identify competitive, noncompetitive, or uncompetitive inhibitors of any MMP-12CD. The invention method works using any mammalian MMP.

Further, other substrates for MMPs may be used, including fluorigenic peptide-2 ("FP-2") and fluorigenic peptide-3 ("FP-3"), which are commercially available.

As shown by Example 5 above, FP-2 may be particularly used in the invention method for identifying competitive, noncompetitive, or uncompetitive inhibitors of TACE (D.E. Van Dyk et. al., *Bioorganic and Medicinal Chemistry Letters,* 1997;7:1219). FP-2 has the sequence Mca-Pro-Leu-Ala-Gln-Ala-Val-Dap(Dnp)-Arg-Ser-Ser-Ser-Arg-NH$_2$, and is available from Bachem Americas (catalog number M-2255).

As mentioned above, the present invention provides a method of identifying competitive, noncompetitive, or uncompetitive inhibitors of metalloenzymes, including inhibitors of MMPs. The method of the present invention works for any enzyme that employs a functional metal cation. All that is required to practice the invention method with a metalloenzyme not described in one of the above examples is to employ an art-recognized assay method for identifying inhibitors of the metalloenzyme, and add, at about the time the substrate of the metalloenzyme is added, a suitable amount of a ligand to a functional metal cation of the metalloenzyme. A suitable amount of the ligand to the functional metal cation is readily determinable by one of ordinary skill in the biochemical arts simply by determining, if necessary, the $K_i$ for the ligand with the metalloenzyme being employed, and employing an amount of ligand as described above.

The present invention improves upon methodology already developed for known assays for identifying inhibitors of metalloenzymes. For example, the invention method provides rapid and easy identification of competitive, noncompetitive, or uncompetitive inhibitors of a metalloenzyme, which identification is useful for identifying pharmaceutical or animal health products. Rapid and easy identification of competitive, noncompetitive, or uncompetitive inhibitors of a metalloenzyme has not, until now, been possible. The invention method avoids laborious and time-consuming enzyme-kinetics experiments for determining whether an inhibitor of a metalloenzyme is competitive, noncompetitive, or uncompetitive. The invention method also avoids using labeled protein or instrumentation not normally found in an enzyme-screening laboratory. The present invention may further be adapted for high throughput screening of compound libraries, which is a valuable drug discovery approach that is widely used in the pharmaceutical and animal health industries.

## Claims

1.  A method for identifying a compound as a competitive, noncompetitive, or uncompetitive inhibitor of an enzyme having a functional metal cation, the method comprising the steps of:

    (a) producing a first control mixture comprising the enzyme, a substrate of the enzyme, and a ligand to the functional metal cation, wherein the first control mixture does not contain the compound;
    (b) producing a second control mixture comprising the enzyme and a substrate of the enzyme, wherein the second control mixture does not contain the compound or the ligand to the functional metal cation;
    (c) producing a first compound mixture comprising the compound, the enzyme, a substrate of the enzyme, and the ligand to the functional metal cation;
    (d) producing a second compound mixture comprising the compound, the enzyme, and a substrate of the enzyme, wherein the second compound mixture does not contain the ligand to the functional metal cation;
    (e) determining inhibition of the enzyme in step (c) by comparing the activity of the enzyme in step (c) to the control activity of the enzyme in step (a);
    (f) determining inhibition of the enzyme in step (d) by comparing the activity of the enzyme in step (d) to the control activity of the enzyme in step (b); and
    (g) comparing the inhibition determined in step (e) with the inhibition determined in step (f).

2.  The method according to Claim 1, wherein the compound is a competitive inhibitor of the enzyme having a functional metal cation.

3.  The method according to Claim 1, wherein the compound is a noncompetitive inhibitor of the enzyme having a functional metal cation.

4.  The method according to Claim 1, wherein the compound is an uncompetitive inhibitor of the enzyme having a functional metal cation.

5.  The method according to Claim 1, wherein the enzyme having a functional metal cation is a matrix metalloproteinase, or a catalytic domain thereof.

6.  The method according to Claim 1, wherein the enzyme having a functional metal cation is MMP-12, or a catalytic domain thereof.

7.  The method according to Claim 1, wherein the enzyme having a functional metal cation is MMP-12 catalytic domain.

8.  The method according to Claim 1, wherein the enzyme having a functional metal cation is MMP-12 catalytic domain, and the ligand to the functional metal cation is 1,10-phenanthroline, or a pharmaceutically acceptable salt thereof.

9.  The method according to Claim 1, wherein the enzyme having a functional metal cation is MMP-13, or a catalytic domain thereof.

10. The method according to Claim 1, wherein the enzyme having a functional metal cation is MMP-13 catalytic domain.

11. The method according to Claim 1, wherein the enzyme having a functional metal cation is MMP-13 catalytic domain, the ligand to the functional metal cation is acetohydroxamic acid, and the inhibitor is a noncompetitive inhibitor.

12. The method according to Claim 1, wherein the ligand to the functional metal cation is acetohydroxamic acid.

13. The method according to Claim 1, further comprising utilizing steady-state kinetics to determine whether the compound is a noncompetitive inhibitor or uncompetitive inhibitor.

# FIG. 1